(19) Europäisches Patentamt
European Patent Office
Office européen des brevets

(11) Veröffentlichungsnummer: **0 087 105**
B1

(12) # EUROPÄISCHE PATENTSCHRIFT

(45) Veröffentlichungstag der Patentschrift: **05.12.84**

(51) Int. Cl.[3]: **C 07 C 33/50**, C 07 C 33/34, A 01 N 31/04, C 07 C 29/36

(21) Anmeldenummer: **83101428.7**

(22) Anmeldetag: **15.02.83**

(54) **Fungizid wirksame Benzhydrolderivate.**

(30) Priorität: **20.02.82 DE 3206225**

(43) Veröffentlichungstag der Anmeldung: **31.08.83 Patentblatt 83/35**

(45) Bekanntmachung des Hinweises auf die Patenterteilung: **05.12.84 Patentblatt 84/49**

(84) Benannte Vertragsstaaten: **BE CH DE FR IT LI NL**

(56) Entgegenhaltungen:
**GB - A - 625 827**
**US - A - 3 287 213**
**US - A - 3 506 682**

**HELVETICA CHIMICA ACTA 37, fasciculus 7, herausgegben am 1. Dezember 1954, Verlag Helvetica Chimica Acta, Seiten 2230-2251, Basel, CH. V. BIRO et al.: "Studien über Konstitution und Wirkung von relativ apolaren, lipoidaffinen Kontaktinsektiziden. 3. Mitteilung. Cyclopropanderivate"**
**CHEMISCHE BERICHTE, Band 109, 1976, Seiten 2185-2196, Verlag Chemie, Weinheim, DE. D. BAUER et al.: "Cyclopropylmethyl-, Äthylcyclopropyl- und Cyclopropylisopropylacetylen- Darstellung und Metallierungsreaktionen"**

(73) Patentinhaber: **Degussa Aktiengesellschaft, Weissfrauenstrasse 9, D-6000 Frankfurt am Main 1 (DE)**

(72) Erfinder: **Schwarze, Werner, Dr. Dipl.-Chem., Leerbachstrasse 117, D-6000 Frankfurt am Main (DE)**
Erfinder: **Kleemann, Axel, Dr. Dipl.-Chem., Greifenhagenstrasse 9, D-6450 Hanau am Main 9 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 105 B1

ACTORUM AG

## Beschreibung

Die Erfindung betrifft neue Benzhydrolderivate, die sich durch eine fungizide Wirksamkeit insbesondere gegen *Piricularia oryzae* in Reis auszeichnen, Verfahren zur Herstellung dieser Verbindungen, Mittel, die diese Verbindungen als Wirkstoffe enthalten, sowie die Verwendung dieser Wirkstoffe als fungizide Mittel im Pflanzenschutz.

Es ist bekannt, dass das Di-(p-chlorphenyl)-cyclopropylmethanol eine fungizide Wirkung gegen *Fusanium culmorum, Alternaria Tenuis, Botrytis cinera* und *Phytophthora infestans* aufweist (US-PS Nr. 3287213). Jedoch werden bei Anwendung dieses Wirkstoffes die Kulturpflanzen oft nachteilig beeinflusst.

Es wurde nun gefunden, dass Benzhydrolderivate der allgemeinen Formel I:

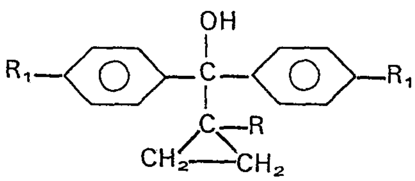 (I)

in der R eine Äthyl- oder vorzugsweise Methylgruppe bedeutet, und $R_1$ für ein Halogen- oder Wasserstoffatom steht, eine ausgezeichnete fungizide Wirkung zum Schutz von Kulturpflanzen, insbesondere gegen *Piricularia oryzae* in Reis aufweisen, ohne die Nutzpflanzen durch unerwünschte Nebenwirkungen nachteilig zu beeinflussen.

Mit den Wirkstoffen der Formel I können an Pflanzen oder Pflanzenteilen (Früchten, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben.

Die Verbindungen der Formel I können ferner als Beizmittel zur Behandlung von Saatgut (Früchten, Knollen, Körnern) und Pflanzenstecklingen zum Schutz von Pilzinfektionen sowie gegen im Erdboden auftretende phytopathogene Pilze eingesetzt werden.

Insbesondere zeichnen sich Verbindungen der allgemeinen Formel I aus, in der $R_1$ für ein Fluor- oder Chloratom steht, und R die Methylgruppe bedeutet. Bevorzugt ist jedoch die Verbindung α-(1-Methylcyclopropyl)-4,4'-dichlorbenzhydrol.

Die erfindungsgemässen Verbindungen können aus den entsprechenden Halogen- bzw. Dihalogenbenzolen und den 1-Methyl- bzw. 1-Äthylcyclopropancarbonsäureestern in an sich bekannter Weise mittels einer Grignard-Reaktion hergestellt werden.

Die genannten Cyclopropylcarbonsäureester sind nach dem von Cannonetal, „Am. Soc.", 81, 81 664 st, beschriebenen Verfahren herstellbar.

Die nachfolgenden Beispiele dienen zur näheren Erläuterung der Erfindung, ohne dieselbe einzuschränken.

*Beispiel 1:*

Man gibt in einen 6-l-Dreihalsrundkolben, der mit einem Rührwerk, einem Gaseinleitungsrohr, Rückflusskühler, Einlauftrichter und Thermometer versehen ist, unter Stickstoff 192 g Magnesiumspäne.

Es wird eine Lösung von 1176 g 1,4-Dichlorbenzol in 2,5 l Tetrahydrofuran bereitet. Von dieser Lösung gibt man etwa 200 ml zu den Magnesiumspänen. Unter Rühren gibt man 10 ml Äthylbromid hinzu. Nach einigen Minuten springt die Reaktion an. Nun tropft man die übrige Dichlorbenzollösung hinzu, so dass die Mischung immer siedet. Anschliessend wird 2 h nacherhitzt.

Zu der erhaltenen Grignard-Lösung tropft man unter Rückfluss 533,5 g 1-Methylcyclopropancarbonsäureäthylester ein. Anschliessend wird noch 2 h unter Rückfluss zum Sieden erhitzt.

Danach kühlt man ab und giesst auf eine Mischung von 2 kg Eis und 500 g Eisessig auf. Die erhaltene Suspension wird mit Toluol extrahiert. Der Extrakt wird mit Natriumsulfat getrocknet und im Vakuum eingedampft. Der ölige Rückstand wird im Hochvakuum destilliert. Bei $Kp._{0,9\,mm}$ 180-182°C destilliert 1-Methylcyclopropylbis-(4-chlorphenyl)carbinol = Bis-(p-chlorphenyl)-1-methylcyclopropylmethanol.

Menge: 822,8 g = 67% d. Th. Gelbes Öl.

*Analyse für* $C_{17}H_{16}Cl_2O$ (Mol-Gew. 307)

| | | | |
|---|---|---|---|
| Berechnet: | C 66,4 | H 5,2 | Cl 23,1% |
| Gefunden: | C 66,2 | H 5,1 | Cl 23,0% |

*Beispiel 2:*

Man löst 70 g 1-Fluor-4-brombenzol in 300 ml Diäthyläther und stellt daraus mit 9,6 g Magnesiumspänen die entsprechende Grignard-Verbindung her. Zu dieser Lösung tropft man unter Rückfluss 26,9 g 1-Methylcyclopropancarbonsäureäthylester. Anschliessend rührt man noch 2 h bei Zimmertemperatur. Dann giesst man auf Eis und neutralisiert mit verdünnter Salzsäure. Die ätherische Lösung wird eingedampft. Es bleibt ein hellbraunes Öl zurück, das im Vakuum destilliert wird. Bei $Kp._{1\,mm}$ 141-143°C destilliert ein fast farbloses Öl.

Menge: 38,5 g, entsprechend 70,25% d. Th.

α-(1-Methylcyclopropyl)-4,4'-difluorbenzhydrol.

*Analyse für* $C_{17}H_{16}OF_2$ (Mol-Gew. 274)

| | | | |
|---|---|---|---|
| Berechnet: | C 74,5 | H 5,8 | F 13,8% |
| Gefunden: | C 74,2 | H 5,7 | F 13,6% |

*Beispiel 3:*

Man löst 94,4 g 1,4-Dibrombenzol in 400 ml Diäthyläther und stellt daraus mit 9,6 g Magnesiumspänen die entsprechende Grignard-Verbindung her. Die Lösung wird anschliessend mit 26,9 g 1-Methylcyclopropancarbonsäureäthylester bei etwa 30°C umgesetzt und anschliessend, wie in Beispiel 2 beschrieben, aufgearbeitet. Das so erhaltene hellrote Öl wird im Vakuum destilliert. Bei $Kp._{0,5\,mm}$ 187-189°C destilliert α-(1-Methylcyclopropyl)-4,4'-dibrombenzhydrol.

Menge: 45,8 g, entsprechend 58,1% d. Th.

*Analyse für* $C_{17}H_{16}OBr_2$ (Mol-Gew. 394)

| | | | |
|---|---|---|---|
| Berechnet: | C 51,8 | H 4,1 | Br 40,6% |
| Gefunden: | C 51,7 | H 4,0 | Br 40,3% |

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen, wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in fungiziden Zubereitungen liegt zwischen 0,1 bis 99, insbesondere zwischen 0,1 bis 95%. Der Gehalt an festen und/oder flüssigen Zusatzstoffen beträgt 1 bis 99,9, vorzugsweise 5 bis 99,9%. Sofern ein Tensid mitverwendet wird, liegt sein Gehalt vorteilhaft zwischen 0,1 bis 25%.

Zur Applikation können die Verbindungen der Formel I in den folgenden Aufarbeitungsformen vorliegen (wobei die Gewichtsprozentangaben in Klammern vorteilhafte Mengen an Wirkstoff darstellen):

*Feste Aufarbeitungsformen:*

Stäube- und Streumittel (bis zu 10%) Granulate, Umhüllungs-, Imprägnierungs- und Homogengranulate, Pellets (Körner) 1 bis 80%.

*Flüssige Aufarbeitungsformen:*

a) in Wasser dispergierbare Wirkstoffkonzentrate:
   Spritzpulver *(wettable powders)* und Pasten (25 bis 90% in der Handelspackung, 0,01 bis 15% in gebrauchsfertiger Lösung);
   Emulsions- und Lösungskonzentrate (10 bis 50%; 0,1 bis 15% in gebrauchsfertiger Lösung);
b) Lösungen (0,1 bis 20%); Aerosole.

Die Wirkstoffe der Formel I vorliegender Erfindung können beispielsweise wie folgt formuliert werden:

*Stäubemittel:*

Zur Herstellung eines a 5%igen und b 2%igen Stäubemittels werden die folgenden Stoffe verwendet:

a) 5 Teile Wirkstoff,
   95 Teile Talkum;
b) 2 Teile Wirkstoff,
   1 Teil hochdisperse Kieselsäure,
   97 Teile Talkum.

Die Wirkstoffe werden mit den Trägerstoffen vermischt und vermahlen und können in dieser Form zur Anwendung verstäubt werden.

*Granulat:*

Zur Herstellung eines 5%igen Granulates werden die folgenden Stoffe verwendet:

5 Teile Wirkstoff,
0,25 Teile epoxidiertes Pflanzenöl,
0,25 Teile Cetylpolyglykoläther,
3,5 Teile Polyäthylenglykol,
91 Teile Kaolin (Korngrösse 0,3 bis 0,8 mm).

Die Aktivsubstanz wird mit dem epoxidierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

*Spritzpulver:*

Zur Herstellung eines a 70%igen, b 40%igen, c und d 25%igen, e 10%igen Spritzpulvers werden folgende Bestandteile verwendet:

a) 70 Teile Wirkstoff,
   5 Teile Natriumdibutylnaphtylsulfonat,
   3 Teile Naphthalinsulfonsäure/Phenolsulfonsäuren/Formaldehyd-Kondensat 3:2:1,
   10 Teile Kaolin,
   12 Teile Champagnekreide;
b) 40 Teile Wirkstoff,
   5 Teile Ligninsulfonsäurenatriumsalz,
   1 Teil Dibutylnaphthalinsulfonsäurenatriumsalz,
   54 Teile Kieselsäure;
c) 25 Teile Wirkstoff,
   4,5 Teile Calciumligninsulfonat,
   1,9 Teile Champagnekreide/Hydroxyäthylcellulose-Gemisch (1:1),
   1,5 Teile Natriumdibutylnaphthalinsulfonat,
   19,5 Teile Kieselsäure,
   19,5 Teile Champagnekreide,
   28,1 Teile Kaolin;
d) 25 Teile Wirkstoff,
   2,5 Teile Isooctylphenoxypolyoxyäthylenäthanol,
   1,7 Teile Champagnekreide/Hydroxyäthylcellulose-Gemisch (1:1),
   8,3 Teile Natriumaluminiumsilikat,
   16,5 Teile Kieselgur,
   46 Teile Kaolin;
e) 10 Teile Wirkstoff,
   3 Teile Gemisch der Natriumsalze von gesättigten Fettalkoholsulfaten,
   5 Teile Naphthalinsulfonsäure/Formaldehyd-Kondensat,
   82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

*Emulgierbare Konzentrate:*

Zur Herstellung eines 25%igen emulgierbaren Konzentrates werden folgende Stoffe verwendet:

25 Teile Wirkstoff,
2,5 Teile epoxidiertes Pflanzenöl,
10 Teile eines Alkylarylsulfonat/Fettalkoholpolyglykoläther-Gemisches,

5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

Die Verbindungen der Formel I können, um sie den gegebenen Umständen anzupassen, selbstverständlich zur Verbreiterung ihres Wirkungsspektrums mit anderen geeigneten Pestiziden oder den Pflanzenwuchs fördernden Wirkstoffen zusammen eingesetzt werden. Als Mischungspartner lassen sich, je nach Anwendungsgebiet, beispielsweise die in der DE-OS Nr. 2506598, S. 6 bis 12, aufgeführten Wirkstoffe verwenden.

*Beispiel 4:*

*Wirkung gegen* Piricularia oryzae *auf Reis*

Reispflanzen werden nach zweiwöchiger Anzucht mit einer aus Spritzpulver des Wirkstoffes hergestellten Spritzbrühe (0,02% Aktivsubstanz) besprüht. Nach 48 h werden die behandelten Pflanzen mit einer Konidiensuspension des Pilzes infiziert. Nach 5 d Inkubation bei 95 bis 100% relativer Luftfeuchtigkeit und 24°C wird der Pilzbefall beurteilt.

Die Verbindung α-(1-Methylcyclopropyl)-4,4'-dichlorbenzhydrol zeigt eine sehr starke pflanzenfungizide Wirkung. Der Krankheitsbefall der behandelten Pflanzen beträgt höchstens 0 bis 5%, was einer 95 bis 100%igen Wirkung entspricht.

Zum Vergleich wird in ganz analoger Weise unter Verwendung der Verbindung Di-(p-chlorphenyl)cyclopropylmethanol als Aktivsubstanz vorgegangen. Es zeigt sich lediglich eine ungenügende Teilwirkung, d.h., der durchschnittliche Krankheitsbefall liegt im Bereich von 20 bis 50%.

## Patentansprüche

1. Benzhydrolderivate der allgemeinen Formel (I):

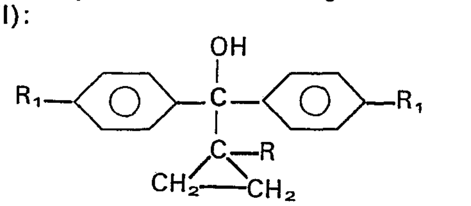

(I)

in der R eine Äthyl- oder vorzugsweise Methylgruppe bedeutet, und $R_1$ für ein Halogen- oder Wasserstoffatom steht.

2. Benzhydrolderivate nach Anspruch 1, dadurch gekennzeichnet, dass in der allgemeinen Formel (I) $R_1$ für ein Fluor- oder Chloratom steht und R die Methylgruppe bedeutet.

3. α-(1-Methylcyclopropyl)-4,4'-dichlorbenzhydrol.

4. Verfahren zur Herstellung von Benzhydrolderivaten nach den Ansprüchen 1 bis 3, dadurch gekennzeichnet, dass man die entsprechenden Halogen- bzw. Dihalogenbenzole mit den 1-Methyl- bzw. 1-Äthylcyclopropancarbonsäureestern, insbesondere -methylestern, in an sich bekannter Weise unter den Bedingungen einer Grignard-Reaktion umsetzt.

5. Fungizides Mittel enthaltend als Wirkstoff eine Verbindung der Formel (I) des Anspruches 1 zusammen mit geeigneten Trägerstoffen und/oder oberflächenaktiven Zusätzen.

6. Verfahren zur Bekämpfung von pflanzenpathogenen Pilzen und Verhütung von Pilzbefall, dadurch gekennzeichnet, dass Pflanzen, Pflanzenteile oder ihre Umgebung mit einer Verbindung der Formel (I) des Anspruches 1 behandelt werden.

7. Verwendung der Verbindung der allgemeinen Formel (I) des Anspruches 1 zur Bekämpfung von pflanzenpathogenen Pilzen und zur Verhütung von Pilzbefall, insbesondere *Piricularia oryzae* in Reis.

## Claims

1. Benzohydrol derivatives corresponding to the general Formula (I):

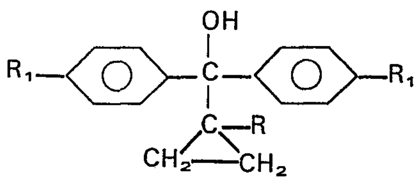

(I)

in which:

R represents an ethyl group or preferably a methyl group, and $R_1$ represents a halogen atom or a hydrogen atom.

2. Benzohydrol derivatives according to Claim 1, characterised in that in the general Formula (I) $R_1$ represents a fluorine or chlorine atom, and R represents the methyl group.

3. α-(1-Methylcyclopropyl)-4,4'-dichlorobenzohydrol.

4. A process for the production of benzohydrol derivatives according to Claims 1 to 3, characterised in that the corresponding halogen- or dihalogenbenzenes are reacted in a known manner with the 1-methyl- or 1-ethylcyclopropane carboxylic acid esters, in particular methyl esters, under the conditions of Grignard's reaction.

5. A fungicide containing as active substance a compound corresponding to Formula (I) of Claim 1 together with suitable carriers and/or surface-active additives.

6. A process for the control of phytopathogenic fungi and for the prevention of a fungus attack, characterised in that plants, parts of plants or their surroundings are treated with a compound corresponding to Formula (I) of Claim 1.

7. The use of the compound corresponding to the general Formula (I) of Claim 1 to control phytopathogenic fungi and to prevent a fungus attack, in particular *Piricularia oryzae* in rice.

## Revendications

1. Dérivés du benzhydrol de formule générale (I):

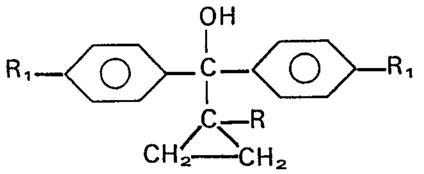

(I)

où R représente un groupe éthyle, ou de préférence un groupe méthyle, et $R_1$ représente un atome d'halogène ou d'hydrogène.

2. Dérivés du benzhydrol selon la revendication 1, caractérisés en ce que, dans la formule générale (I), $R_1$ représente un atome de fluor ou de chlore et R représente un groupe méthyle.

3. α-(1-Méthylcyclopropyl)-4,4'-dichlorobenzhydrol.

4. Procédé d'obtention des dérivés du benzhydrol selon les revendications 1 à 3, caractérisé en ce que l'on fait réagir les halogéno- ou dihalogénobenzènes correspondants avec un ester 1-méthyl- ou 1-éthylcyclopropanecarboxylique, en particulier avec un ester méthylique, de manière connue en soi, selon une réaction de Grignard.

5. Agent fongicide contenant comme matière active un composé répondant à la formule (I) de la revendication 1, en combinaison avec des supports appropriés et/ou des additifs tensio-actifs.

6. Procédé pour lutter contre les champignons pathogènes des végétaux et pour prévenir les infections par les champignons, caractérisé en ce que les végétaux, leurs parties constitutives ou leur environnement sont traités avec un composé de la formule (I) de la revendication 1.

7. Application des composés de formule générale (I) selon la revendication 1, pour lutter contre les champignons pathogènes des plantes et pour éviter les infections par ces champignons, en particulier par le *Piricularia oryzae* sur le riz.